# EUROPEAN PATENT SPECIFICATION

(11) **EP 0 614 342 B1**
(45) Date of publication and mention of the grant of the patent: **14.07.1999**
(21) Application number: 92925402.7
(22) Date of filing: 25.11.1992
(51) Int. Cl.: A61B 17/12, A61B 17/00

(54) **CLOSURE PROSTHESIS FOR TRANSCATHETER PLACEMENT**
VERSCHLUSSPROTHESE FÜR DIE KATHETERPLAZIERUNG
PROTHESE DE FERMETURE A METTRE EN PLACE PAR L'INTERMEDIAIRE D'UN CATHETER

(30) Priority: 29.11.1991 US 800439
(43) Date of publication of application: 14.09.1994
(73) Proprietor: WILLIAM COOK EUROPE A/S, 4632 Bjaeverskov (DK); PAVCNIK, Dusan, SI-65000 Nova Gorica (SI); WALLACE, Sidney, Houston, TX 77005 (US); WRIGHT, Kenneth C., Houston, TX 77006 (US)
(72) Inventor: PAVCNIK, Dusan, SI-65000 Nova Gorica (SI); WALLACE, Sidney, Houston, TX 77005 (US); WRIGHT, Kenneth C., Houston, TX 77006 (US)
(74) Representative: Bannerman, David Gardner
(86) International application number: US9210141
(87) International publication number: WO9310714

(56) References cited:
- US-A- 3 874 388
- US-A- 4 007 743

## Description

### BACKGROUND OF THE INVENTION

This invention relates in general to closure prostheses which might find application for transcatheter placement within the heart to close congenital and operative cardiovascular defects.

Examples of such cardiovascular defects include those which result in an abnormal opening in the cardiovascular system and permit deleterious shunting of blood thereacross, such as atrial and ventricle septum defects and patent ductus arteriosus. To minimize the risk of mortality associated with surgery, transcatheter techniques have been developed to introduce closure devices for sealing such defects. One example of such a device is described in Blake, U.S. Patent No. 4,007,743. Blake discloses a pair of umbrella-like closure devices which include resilient ring sections to automatically open the struts of the umbrella as the devices are pushed out of their respective catheters. King et al., U.S. Patent No. 3,874,388 upon which the preamble of claim 1 is based, similarly discloses a pair of umbrella-like closure devices which interconnect after placement to seal across a defect.

These and other such devices rely on the caudal and cranial ends of the device being larger than the opening of the defect itself to physically trap the device across the opening. To accommodate transcatheter delivery techniques, the resulting device configurations have become unduly mechanical in nature, often including multiple components or requiring sequential delivery. Also, many of these devices require assembly across the defect after delivery, thereby increasing the complexity of the transcatheter equipment and delivery process.

A need therefore exists for an improved closure device. Such a device would be of a compact, unitary construction, therefore permitting the device to be delivered intact using transcatheter techniques. Also, such a device would be delivered from a sheath or catheter in a one-step procedure, rather than requiring sequential delivery. Similarly, such a device would be manually controlled after delivery to ensure proper placement and sealing across the defect. Preferably, the device would also be self-centering to facilitate proper delivery and placement across the defect.

### SUMMARY OF THE INVENTION

The invention provides a flexible closure prosthesis and transcatheter delivery system for sealing an opening with a diameter in a fluid passageway within a living body, comprising:
a hollow sheath sized to fit through the opening and having a proximal end;
a pusher catheter slidable within said sheath;
at least one filament slidably connected to the closure prosthesis and having ends extending proximally from said proximal end of said sheath; and
a flexible sealing element that is capable of being compressed into a reduced insertion form suitable for insertion into said hollow sheath for delivery adjacent the opening but tending to assume a relatively thin shape with a surface area sufficiently large to cover the opening, characterised by:
at least one flexible restraining element, fixedly attached to and extending across one side of said sealing element;
said at least one filament slidably connected to said at least one restraining element; and
said restraining element being capable of being deformed by said at least one filament into a reduced insertion form suitable for insertion through the opening but tending to assume a configuration lying against said one side of said sealing element such that the closure prosthesis is pinching an edge of the opening between a portion of said restraining element and said sealing element.

One object of the present invention is to provide an improved closure prosthesis and delivery system adapted thereto.

Another object of the present invention is to provide a closure prosthesis having a compact, unitary construction permitting the device to be delivered intact using transcatheter techniques.

Another object of the present invention is to provide a closure prosthesis which is delivered from a sheath or catheter using a one-step procedure.

Another object of the present invention is to provide a closure prosthesis which can be manually controlled after delivery to ensure proper placement and sealing across the defect.

Still another object of the present invention is to provide a closure prosthesis which is self-centering.

Related objects and advantages of the present invention will become apparent from the following description.

### BRIEF DESCRIPTION OF THE DRAWINGS

FIG. 1 is a rear elevational view of a disk prosthesis according to one embodiment of the present invention.
FIG. 2 is a side elevational view of the disk prosthesis shown in FIG. 1.
FIG. 3 is an elevational view of a flexible ring of a disk prosthesis having flexible elements attached thereto according to another embodiment of the present invention.
FIG. 4 is a side elevational view of a pusher catheter connected by filaments to the disk prosthesis of FIG. 1 according to another embodiment of the present invention.
FIG. 5 is a side elevational view of an introducer sheath assembly containing the pusher catheter and disk prosthesis of FIG. 4 therein.
FIG. 6 is an exploded cross-sectional view of the distal end of the sheath assembly of FIG. 5.
FIG. 7 is a cross-sectional view of the pusher catheter of FIG. 4 and including a wire guide according to another embodiment of the present invention.
FIG. 8 is a partial cross-sectional view of the distal end of the introducer sheath assembly of FIG. 5 inserted and extending through a cardiovascular defect.
FIG. 9 is a partial cross-sectional view of the distal end of the pusher catheter of FIG. 4 inserted and extending through a cardiovascular defect with the disk prosthesis drawn tight against the catheter.
FIG. 10 is a partial cross-sectional view of the distal end of the pusher catheter of FIG. 4 adjacent a cardiovascular defect with the disk prosthesis seated against the vessel walls of the defect.
FIG. 11 is a partial cross-sectional view of the disk prosthesis of FIG. 1 in place and sealing across a cardiovascular defect.

### DESCRIPTION OF THE PREFERRED EMBODIMENT

For the purposes of promoting an understanding of the principles of the invention, reference will now be made to the embodiments illustrated in the drawings and specific language will be used to describe the same. It will nevertheless be understood that no limitation of the scope of the invention is thereby intended, such alterations and further modifications in the illustrated device, and such further applications of the principles of the invention as illustrated therein being contemplated as would normally occur to one skilled in the art to which the invention relates.

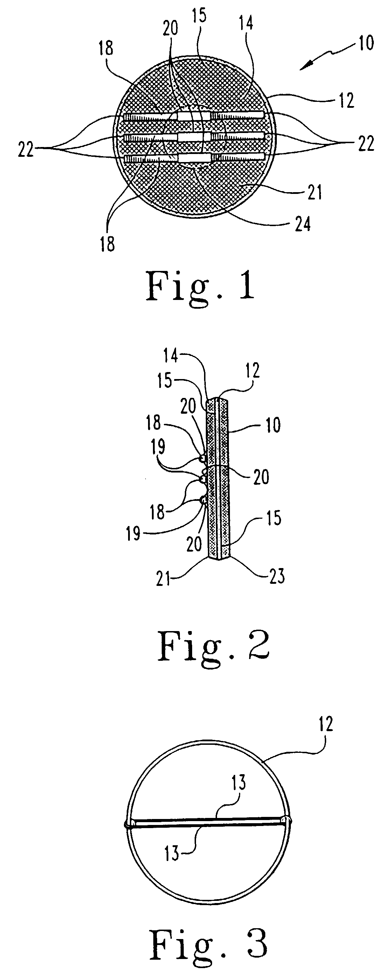

Referring now to FIGS. 1 and 2, a flexible disk 10 is shown including a flexible ring 12 covered by diaphragm 14. Disk 10 is resiliently compressible so as to flex when restrained into a smaller form to facilitate insertion and placement thereof, wherein when compressed disk 10 resiliently bends without permanent deformation to store energy therein. Unrestrained, disk 10 resiliently expands into a larger operational form by releasing the energy stored therein, wherein disk 10 resiliently unbends without permanent deformation to its original form.

Diaphragm 14 attaches to ring 12 and functions to seal across ring 12. Therefore, flexible ring 12 and diaphragm 14 attached thereto define a sealing element sized to seal across an opening of a cardiovascular defect. Other shaped flexible sealing elements in addition to disk shapes are contemplated as well, wherein the sealing element conforms to the shape of the defect. Similarly, other constructions of sealing elements are contemplated, such as a flexible ring with only one diaphragm or an integral ring and diaphragm constructed of a resiliently flexible material.

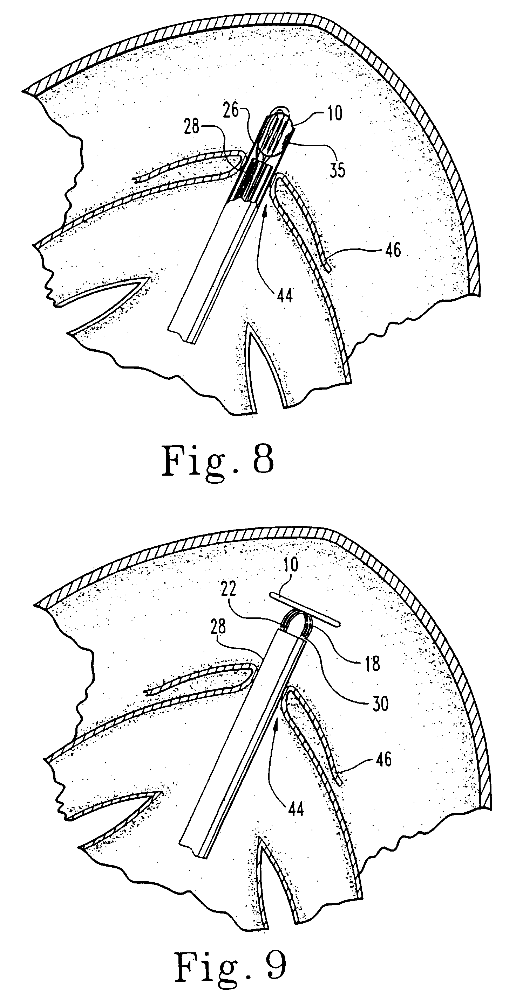

Attached to diaphragm 14 are flexible wire segments 18 for trapping the disk in place. As such, flexible wire segments 18 define a restraining element sized to restrain the disk across the defective opening. Flexible ring 12 is sized to a diameter larger than that of the opening, and wire segments 18 are similarly of a length greater than the defect diameter. Wire segments 18 are hollow cylinders and include openings 19 extending longitudinally therethrough. Wire segments 18 are attached along a portion of their length to the central portion of diaphragm 14 at attachment points 20, thereby defining free ends 22 of wire segments 18 at either side of the attachment points which are detached from diaphragm 14 and free to flex outwardly from disk 10. Attachment points 20 of wire segments 18 to diaphragm 14 collectively define an attachment zone 24, as depicted by the dashed lines in FIG. 1. Because the free ends of wire segments 18 are pulled outwardly from the disk into a smaller form to pass through the defect opening during deployment as shown in FIGS. 8 through 11, attachment zone 24 should be sized smaller than the defect opening. Other flexible restraining element configurations in addition to flexible wire segments are contemplated as well, such as overlapping flexible tabs or ears with openings therethrough which when resiliently bent into a smaller form define an attachment zone sufficiently small to pass through a defect opening.

In one embodiment of the present invention, flexible ring 12 is constructed of (0.064-0.071cm) (0.025 to 0.028 inch) diameter stainless steel coiled wire formed into a ring and having an overall diameter of 2 cm. As such, in this embodiment disk 10 is capable of sealing an opening of up to 2 cm. in diameter; however, to provide a margin of safety, in this embodiment disk 10 seals an opening of approximately 1.5 cm. in diameter. Ring 12 is covered by a double wall nylon mesh material including nylon meshes 21 and 23 containing a nylon foam core 15 within ring 12 to define diaphragm 14, wherein the nylon mesh is threaded onto the ring to attach thereto to trap the foam core in place and the foam core functions to substantially seal across ring 12.

Three lengths of (0.081-0.089cm) (0.032 to 0.035 inch) diameter stainless steel coiled wire are attached along the mid-portions of their lengths to diaphragm 14. Attachment techniques common to the industry are employed depending on the size and operational characteristics of the disk to be deployed. For example, wire segments 18 can be looped or threaded through a portion of the nylon mesh forming diaphragm 14. In another embodiment depicted in FIG. 3, flexible ring 12 includes flexible elements 13 for added structure and resiliency. Flexible elements 13 are formed by attaching two lengths of elastic filament to ring 12. In this embodiment, the elastic filaments are constructed of rubber or an equivalent material and are tied to ring 12 using nylon filament ties. Ring 12 further includes two to three loops (0.025cm) (0.010inch) diameter stainless steel safety wire inside its (0.064-0.071 cm) (0.025 to 0.028 inch) diameter for added resiliency to maintain its circular shape.

Multiple foam cores are contemplated on either side of flexible elements 13, wherein a nylon mesh is similarly attached to ring 12 to trap the foam cores in place to substantially seal across ring 12. Fasteners and adhesives may also be employed either to attach the wire segments to the diaphragm or to provide a redundant attachment for improved margins of safety over existing attachments.

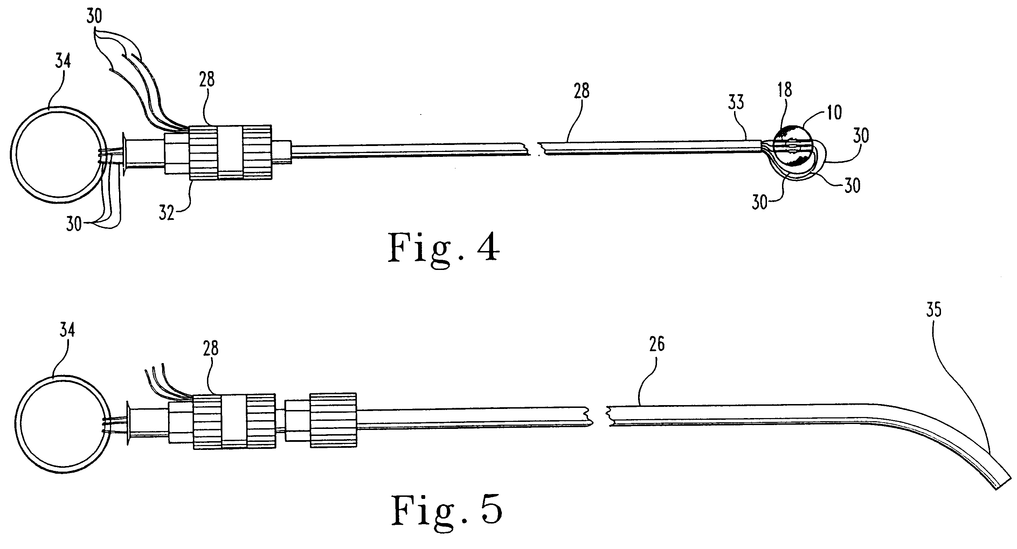

As shown in FIGS 4 and 5, disk 10 is capable of percutaneous insertion via an introducer sheath assembly 26 and employing a pusher catheter 28. Sheath assembly 26 is adapted to insert into the femoral vein, extending into the heart and providing a pathway for positioning disk 10 adjacent a defect. Delivery of the sheath may be accomplished according to standard medical procedures. One delivery scheme might include inserting a wire guide through a needle and positioning the wire guide adjacent the defect. The sheath assembly with a dilator can be inserted over the wire guide, wherein the wire guide and dilator are then removed leaving the sheath assembly in place. In one embodiment, a 3mm (9 F) sheath assembly is used in conjunction with a 2.33mm (7 F) pusher catheter, wherein the sheath assembly is 80 cm. long and the pusher catheter is slightly longer at 81 cm.

Disk 10 is resiliently collapsible along the longitudinal axis of wire segments 18 into a smaller cross-sectional shape for insertion within sheath assembly 26. As depicted in FIG. 4, disk 10 is connected to pusher catheter 28 via filaments 30 prior to insertion within sheath assembly 26. A number of filament lengths greater than or equal to the number of wire segments 18 of disk 10 are used, wherein each length of filament is sufficiently long and of reduced diameter to pass through catheter 28 and wire segments 18. In one embodiment, filaments 30 are (2 × 10⁻³ cm) (0.008 inch) diameter nylon filament, each approximately 3 meters in length. Each of filaments 30 are attached at one of their ends to proximal end 32 of catheter 28. Each filament is routed through and exits the distal end 33 of catheter 28, through openings 19 of wire segments 18, and returns through catheter 28 to attach to ring 34. Ring 34 is larger than catheter 28 to prevent filaments 30 from retracting into the catheter. As such, ring 34 will abut catheter 28 when released to prevent slackened filaments 30 from retracting into the catheter. Conversely, pulling ring 34 away from catheter 28 takes up slack in filaments 30 until disk 10 abuts the distal end 33 of catheter 28. Further pulling of ring 34 tightens filaments 30 and pulls free ends 22 of wire segments 18 away from disk 10.

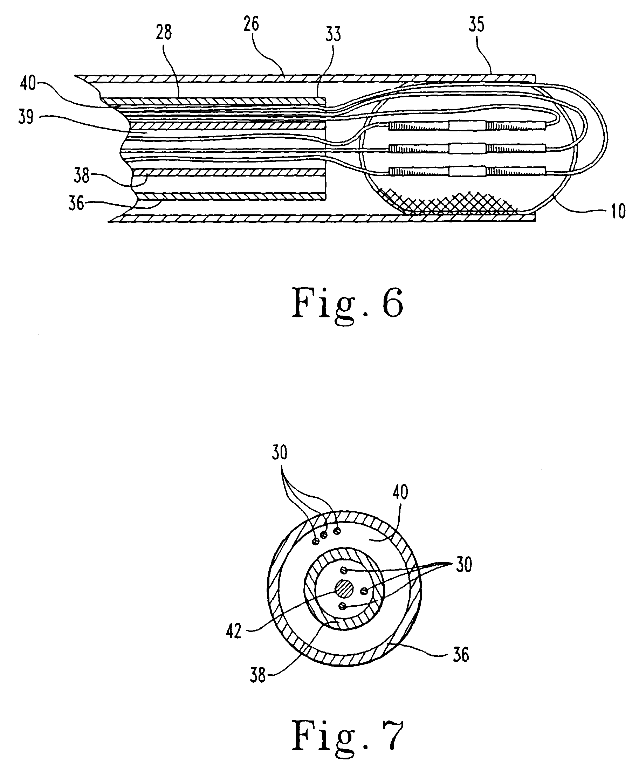

Referring now to FIG. 6, an exploded cross-sectional view of the distal end 33 of catheter 28 is shown, with disk 10 connected thereto by filaments 30 and compressed within the distal end 35 of sheath assembly 26 prior to delivery. Catheter 28 is a double-lumen catheter including an inner catheter 38 and an outer catheter 36. The bore of inner catheter 38 defines inner lumen 39, wherein the annulus formed between inner catheter 38 and outer catheter 36 defines outer lumen 40. In one embodiment, the inner catheter is a (1.6mm) (5 F) catheter and the outer catheter is a (2.33mm) (7 F) catheter. Filaments 30 pass through outer lumen 40 as they extend from the proximal end 32 of catheter 28 through to wire segments 18 of disk 10. Filaments 30 return through inner lumen 39 to exit therefrom and attach to ring 34. Therefore, as shown in FIG. 7, filaments 30 are separated from each other by inner catheter 38 as they extend in opposing directions through catheter 28, thereby reducing risk of entanglement. Nevertheless, catheter 28 is also contemplated as a single lumen catheter to permit further reductions in the size of the transcatheter delivery system. Also contemplated is a wire guide 42 disposed within inner catheter 38 to aid in maintaining the inner lumen open, as shown in FIG. 7.

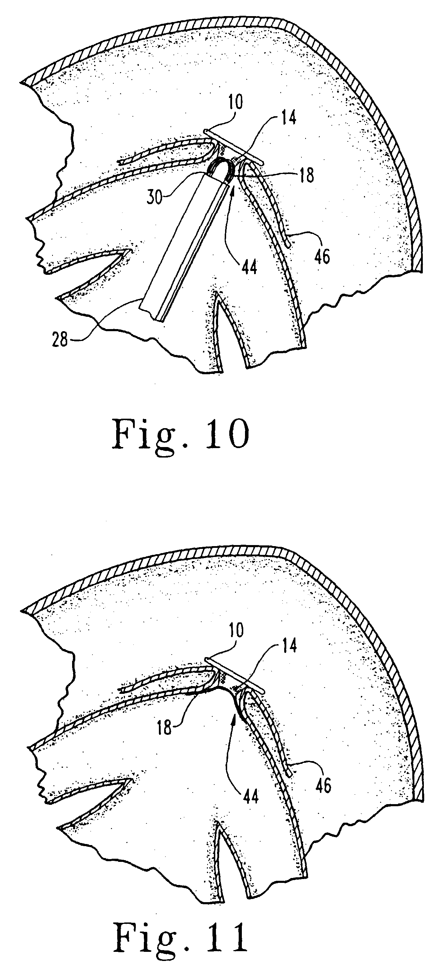

Referring now to FIGS. 8 through 11, the delivery of flexible sealing element (disk 10) is depicted adapted to closing a defect such as a patent ductus arteriosus. In FIG. 8, sheath assembly 26 is shown with its distal end 35 projecting through defect opening 44, with disk 10 resiliently compressed into a first (reduced) insertion form and constrained by the sheath assembly prior to delivery. Disk 10 is delivered by pulling out sheath assembly 26 while holding catheter 28 in place and, as the sheath assembly is withdrawn to expose and release the disk, disk 10 resiliently self-expands to its unrestrained larger operational form. As discussed previously, ring 34 is then pulled outwardly from catheter 28 to take up slack in filaments 30. Due to this motion, disk 10 will abut the distal end of catheter 28 at one or more free ends 22 of wire segments 18, at which point further tightening of filaments 30 results in the wire segments being resiliently compressed and pulled away from disk 10. As such, a second insertion forms is defined permitting the wire segments to be withdrawn through to the side of the defect opposite the disk as shown in FIGS. 9 and 10. As shown in FIG. 10, after the wire segments are withdrawn through the defect, catheter 28 is pulled back from the defect, further tightening filaments 30, to effectively seat the disk against vessel walls 46.

After the disk is in place and seated, the filaments are released from the proximal end 32 of catheter 28 and pulled completely free of the catheter by further extending ring 34. When the filaments are released, wire segments 18 resiliently self-expand against the vessel walls into a second larger operational form for restraining disk 10 across the defect.

As the wire segments 18 expand, diaphragm 14 is pulled away from disk 10 and partially through defect opening 44, thereby exerting a compressive force which traps and centers disk 10 across opening 44. As such, disk 10 self-centers across opening 44 to ensure its proper placement.

## Claims

1. A closure prosthesis and transcatheter delivery system for sealing an opening (44) with a diameter in a fluid passageway within a living body, comprising:
a hollow sheath (26) sized to fit through the opening and having a proximal end;
a pusher catheter (28) slidable within said sheath; at least one filament (30) slidably connected to the closure prosthesis and having ends extending proximally from said proximal end of said sheath (26); and
a flexible sealing element (10) that is capable of being compressed into a reduced insertion form suitable for insertion into said hollow sheath (26) for delivery adjacent the opening (44) but tending to assume a relatively thin shape with a surface area sufficiently large to cover the opening (44), characterised by:
at least one flexible restraining element (18), fixedly attached to and extending across one side of said sealing element (10);
said at least one filament (30) slidably connected to said at least one restraining element (18); and
said restraining element (18) being capable of being deformed by said at least one filament (30) into a reduced insertion form suitable for insertion through the opening (44) but tending to assume a configuration lying against said one side of said sealing element (10) such that the closure prosthesis is pinching an edge of the opening (44) between a portion of said restraining element (18) and said sealing element (10).

2. The closure prosthesis and transcatheter delivery system of claim 1 wherein said at least one flexible restraining element (18) is at least one hollow cylinder; and
said at least one filament (30) is threaded through said hollow cylinder.

3. The closure prosthesis and transcatheter delivery system of claim 1 wherein said sealing element (10) is disk shaped.

4. The closure prosthesis and transcatheter delivery system of claim 1 wherein said sealing element (10) has a center portion (24); and
said at least one flexible restraining element (18) is attached to said center portion (24) of said sealing element (10).

5. The closure prosthesis and transcatheter delivery system of claim 1 wherein each of said at least one flexible restraining element (18) is longer than the diameter of the opening (44).

6. The closure prosthesis and transcatheter delivery system of claim 1 wherein said sealing element (10) comprises a resiliently flexible ring (12) and a flexible diaphragm (14) attached across said ring.

7. The closure prosthesis and transcatheter delivery system of claim 1 wherein said sealing element (10) tends to resiliently assume a planar configuration.

8. The closure prosthesis and transcatheter delivery system of claim 1 wherein said pusher catheter (28) has two lumens (36,38); and
each end of said at least one filament (30) is threaded through a different one of said two lumen (36,38).

9. The closure prosthesis and transcatheter delivery system of claim 1 wherein said flexible sealing element (10) comprises a flexible ring (12) having an opening therethrough and a flexible diaphragm attached to said ring and covering said opening (14).

10. The flexible closure prosthesis and transcatheter delivery system of claim 9 wherein said flexible ring further comprises a plurality of flexible elements (13) attached across said ring.

11. The flexible closure prosthesis and transcatheter delivery system of claim 10 wherein:
said flexible ring (12) is constructed from a length of coiled wire resiliently bent into a ring and attached at its ends and includes two to three loops of flexible safety wire inside said coiled wire;
said flexible elements (13) are constructed of elastic filaments tied across said coiled wire;
said flexible diaphragm (14) is constructed from a double wall nylon mesh (21,23) attached to said flexible ring (12) and containing a flexible foam core therein (15); and
said at least one flexible restraining element (18) is a discrete length of coiled wire, attached along a portion of its length to said diaphragm.

12. The flexible closure prosthesis and transcatheter delivery system of claim 9 wherein:
said flexible ring (12) is constructed from a length of coiled wired resiliently bent into a ring and attached at its ends and includes two to three loops of flexible safety wire inside said coiled wire;
said flexible diaphragm (14) is constructed from a double wall nylon mesh (21,23) attached to said flexible ring and containing a flexible foam core therein (15); and
said at least one flexible restraining elements (18) is a discrete length of coiled wire, attached along a portion of its length to said diaphragm (14).

13. The flexible closure prosthesis and transcatheter delivery system of claim 9 wherein said at least one filament (30) comprises:
a pull ring (34), said pull ring (34) being larger in diameter than said pusher catheter (28) to prevent passage therethrough;
said at least one flexible restraining element (18) including an opening and said filament (30) routed through the opening;
said filament (30) connected at one end to said pusher catheter (28) and at another end to said pull ring (43); and
wherein pulling of said pull ring (34) causes said filaments (30) to deform said restraining element (18).

14. The flexible closure prosthesis and transcatheter delivery system of claim 13 wherein said filaments (30) are monofilaments constructed of nylon.

## Patentansprüche

1. Verschlußprothese zum Verschließen einer einen Durchmesser aufweisenden Öffnung (44) in einem Flüssigkeitskanal im Inneren eines lebenden Organismus und Transkathetersystem zum Einsetzen dieser, mit
einer hohlen Hülse (26), die so dimensioniert ist, daß sie durch die Öffnung paßt, und die ein proximales Ende aufweist,
einem Pusherkatheter (28), der in der Hülse verschiebbar ist,
wenigstens einem Faden (30), der verschiebbar mit der Verschlußprothese verbunden ist und der Enden aufweist, die von dem proximalen Ende der Hülse (26) aus in proximaler Richtung verlaufen, und
einem flexiblen Verschlußelement (10), das sich zu einer verkleinerten Einsetzform komprimieren läßt, in der es in die hohle Hülse (26) einsetzbar ist, so daß es sich bis zu der Öffnung (44) hin zuführen läßt, das jedoch eine relativ dünne Form anzunehmen bestrebt ist mit einer ausreichend großen Oberfläche, so daß die Öffnung (44) bedeckt wird, dadurch gekennzeichnet, daß
wenigstens ein flexibles Halteelement (18) an einer Seite des Verschlußelements (10) festgelegt ist und sich über diese erstreckt,
der wenigstens eine Faden (30) verschiebbar mit dem wenigstens einen Halteelement (18) verbunden ist, und
das Halteelement (18) mittels des wenigstens einen Fadens (30) verformbar ist zu einer verkleinerten Einsetzform, in der es sich durch die Öffnung (44) hindurch einsetzen läßt, es jedoch eine Konfiguration anzunehmen bestrebt ist, in der es an der einen Seite des Verschlußelements (10) anliegt, so daß die Verschlußprothese einen Rand der Öffnung (44) zwischen einem Abschnitt des Halteelements (18) und dem Verschlußelement (10) festklemmt.

2. Verschlußprothese und Transkathetersystem zum Einsetzen dieser nach Anspruch 1, wobei das wenigstens eine flexible Halteelement (18) wenigstens ein Hohlzylinder ist, und
der wenigstens eine Faden (30) durch den Hohlzylinder hindurchgezogen ist.

3. Verschlußprothese und Transkathetersystem zum Einsetzen dieser nach Anspruch 1, wobei das Verschlußelement (10) scheibenförmig ist.

4. Verschlußprothese und Transkathetersystem zum Einsetzen dieser nach Anspruch 1, wobei das Verschlußelement (10) einen Mittelabschnitt (24) aufweist, und
das wenigstens eine flexible Halteelement (18) an dem Mittelabschnitt (24) des Verschlußelements (10) befestigt ist.

5. Verschlußprothese und Transkathetersystem zum Einsetzen dieser nach Anspruch 1, wobei die Länge des wenigstens einen flexiblen Halteelements (18) jeweils größer ist als der Durchmesser der Öffnung (44).

6. Verschlußprothese und Transkathetersystem zum Einsetzen dieser nach Anspruch 1, wobei das Verschlußelement (10) einen elastisch flexiblen Ring (12) und eine auf dem Ring befestigte flexible Membran (14) aufweist.

7. Verschlußprothese und Transkathetersystem zum Einsetzen dieser nach Anspruch 1, wobei das Verschlußelement (10) bestrebt ist, elastisch eine ebene Konfiguration einzunehmen.

8. Verschlußprothese und Transkathetersystem zum Einsetzen dieser nach Anspruch 1, wobei der Pusherkatheter (28) zwei Lumen (36, 38) aufweist, und
jedes Ende des wenigstens einen Fadens (30) durch ein jeweils anderes der beiden Lumen (36, 38) hindurchgeführt ist.

9. Verschlußprothese und Transkathetersystem zum Einsetzen dieser nach Anspruch 1, wobei das flexible Verschlußelement (10) einen flexiblen Ring (12) mit einer durch diesen hindurchgehenden Öffnung und eine flexible Membran aufweist, die an dem Ring befestigt ist und die Öffnung (14) bedeckt.

10. Flexible Verschlußprothese und Transkathetersystem zum Einsetzen dieser nach Anspruch 9, wobei der flexible Ring ferner eine Mehrzahl von flexiblen Elementen (13) aufweist, die über den Ring hinweg befestigt sind.

11. Flexible Verschlußprothese und Transkathetersystem zum Einsetzen dieser nach Anspruch 10, wobei
der flexible Ring (12) aus einem Abschnitt Wendeldraht ausgebildet ist, der elastisch zu einem Ring gebogen und an seinen Enden befestigt ist, und zwei oder drei Schleifen flexiblen Sicherheitsdrahts im Inneren des Wendeldrahts aufweist,
die flexiblen Elemente (13) aus elastischen Fäden ausgebildet sind, die über den Wendeldraht hinweg befestigt sind,
die flexible Membran (14) aus einem doppelwandigen Nylongeflecht (21, 23) ausgebildet ist, das an dem flexiblen Ring (12) befestigt ist und einen flexiblen Schaumstoffkern (15) in seinem Inneren aufweist, und
das wenigstens eine flexible Halteelement (18) ein einzelner Abschnitt Wendeldraht ist, der entlang eines Abschnitts seiner Länge an der Membran befestigt ist.

12. Flexible Verschlußprothese und Transkathetersystem zum Einsetzen dieser nach Anspruch 9, wobei
der flexible Ring (12) aus einem Abschnitt Wendeldraht ausgebildet ist, der elastisch zu einem Ring gebogen und an seinen Enden befestigt ist, und zwei oder drei Schleifen flexiblen Sicherheitsdrahts im Inneren des Wendeldrahts aufweist,
die flexible Membran (14) aus einem doppelwandigen Nylongeflecht (21, 23) ausgebildet ist, das an dem flexiblen Ring befestigt ist und einen flexiblen Schaumstoffkern (15) in seinem Inneren aufweist, und
das wenigstens eine flexible Halteelement (18) ein einzelner Abschnitt Wendeldraht ist, der entlang eines Abschnitts seiner Länge an der Membran (14) befestigt ist.

13. Flexible Verschlußprothese und Transkathetersystem zum Einsetzen dieser nach Anspruch 9, wobei der wenigstens eine Faden (30)
einen Zugring (34) aufweist, wobei der Zugring (34) einen größeren Durchmesser als der Pusherkatheter (28) hat, so daß ein Durchrutschen durch diesen verhindert wird,
das wenigstens eine flexible Halteelement (18) eine Öffnung aufweist und der Faden (30) durch die Öffnung hindurchgeführt ist,
der Faden (30) an einem Ende mit dem Pusherkatheter (28) und an einem anderen Ende mit dem Zugring (34) verbunden ist, und
wobei durch Ziehen an dem Zugring (34) bewirkt wird, daß die Fäden (30) das Halteelement (18) verformen.

14. Flexible Verschlußprothese und Transkathetersystem zum Einsetzen dieser nach Anspruch 13, wobei die Fäden (30) aus Nylon ausgebildete Einzelfäden sind.

## Revendications

1. Prothèse d'obturation et système de mise en place par transcathéter pour étanchéifier une ouverture (44) d'un certain diamètre dans in trajet de passage de fluide à l'intérieur d'un corps vivant, comprenant :
une gaine creuse (26) dimensionnée de façon à s'ajuster à travers l'ouverture et ayant une extrémité proximale ;
un cathéter pousseur (28) pouvant coulisser à l'intérieur de ladite gaine ;
au moins un filament (30) connecté à la prothèse d'obturation de façon à pouvoir coulisser et présentant des extrémités s'étendant proximalement à partir de l'extrémité proximale de ladite gaine (26) ; et
un élément d'étanchéité souple (10) qui est susceptible d'être comprimé sous une forme d'introduction réduite permettant son introduction dans ladite gaine creuse (26) pour être libéré au voisinage de l'ouverture (44) mais tendant à prendre une forme relativement mince avec une aire de surface suffisamment importante pour couvrir l'ouverture (44),
caractérisés en ce que :
au moins un élément de retenue souple (18) est attaché de façon fixe audit élément d'étanchéité (10) en s'étendant au travers d'un de ses côtés;
ledit au moins un filament (30) est connecté de façon à pouvoir coulisser par rapport à lui à au moins un dit élément de retenue (18); et
ledit élément de retenue (18) est susceptible d'être déformé par ledit au moins un filament (30) sous une forme d'introduction réduite permettant son introduction à travers l'ouverture (44) mais tendant à prendre une configuration dans laquelle il se place contre ledit un côté dudit élément d'étanchéité (10) de telle façon que la prothèse d'obturation pince un bord de l'ouverture (44) entre une partie dudit élément de retenue (18) et ledit élément d'étanchéité (10).

2. Prothèse d'obturation et système de mise en place par transcathéter selon la revendication 1, dans lesquels ledit au moins un élément de retenue souple (18) est au moins un cylindre creux ; et
ledit au moins un filament (30) est enfilé à travers ledit cylindre creux.

3. Prothèse d'obturation et système de mise en place par transcathéter selon la revendication 1, dans lesquels ledit élément d'étanchéité (10) a une forme de disque.

4. Prothèse d'obturation et système de mise en place par transcathéter selon la revendication 1, dans lesquels ledit élément d'étanchéité (10) comporte une partie centrale (24) ; et
ledit au moins un élément souple de retenue (18) est fixé à ladite partie centrale (24) dudit élément d'étanchéité (10).

5. Prothèse d'obturation et système de mise en place par transcathéter selon la revendication 1, dans lesquels ledit au moins un élément de retenue souple (18) est plus long que le diamètre de l'ouverture (44).

6. Prothèse d'obturation et système de mise en place par transcathéter selon la revendication 1, dans lesquels ledit élément d'étanchéité (10) comprend une bague souple élastique (12) et un diaphragme souple (14) fixé au travers de ladite bague.

7. Prothèse d'obturation et système de mise en place par transcathéter selon la revendication 1, dans lesquels ledit élément d'étanchéité (10) tend à prendre élastiquement une configuration plane.

8. Prothèse d'obturation et système de mise en place par transcathéter selon la revendication 1, dans lesquels ledit cathéter pousseur (28) comporte deux passages (36, 38) ; et
chaque extrémité dudit au moins un filament (30) est enfilé à travers chacun desdits deux passages (36, 38).

9. Prothèse d'obturation et système de mise en place par transcathéter selon la revendication 1, dans lesquels ledit élément d'étanchéité souple (10) comprend une bague souple (12) présentant une ouverture à travers elle et un diaphragme souple fixé à ladite bague et couvrant ladite ouverture (14).

10. Prothèse d'obturation souple et système de mise en place par transcathéter selon la revendication 9, dans lesquels ladite bague souple comprend en outre une pluralité d'éléments souples (13) fixés en travers de ladite bague.

11. Prothèse d'obturation souple et système de mise en place par transcathéter selon la revendication 10, dans lesquels :
ladite bague souple (12) est constituée par une longueur d'un fil métallique enroulé replié élastiquement sous la forme d'une bague et fixée à ses extrémités et comprend deux à trois boucles de fil métallique souple de sécurité à l'intérieur dudit fil métallique enroulé ;
lesdits éléments souples (13) sont constitués de filaments élastiques attachés en travers dudit fil métallique enroulé ;
ledit diaphragme souple (14) est constitué d'une double paroi à maillage en nylon (21, 23) fixée à ladite bague souple (12) et contenant intérieurement un noyau de mousse souple (15) ; et
ledit au moins un élément de retenue souple (18) est constitué par une longueur discrète d'un fil métallique enroulé fixé le long d'une partie de sa longueur audit diaphragme.

12. Prothèse d'obturation souple et système de mise en place par transcathéter selon la revendication 9, dans lesquels :
ladite bague souple (12) est constituée d'une longueur d'un fil métallique enroulé recourbé élastiquement sous forme d'une bague et fixée à ses extrémités et comprend deux à trois boucles de fil métallique de sécurité souple à l'intérieur dudit fil métallique enroulé ;
ledit diaphragme souple (14) est constitué d'une double paroi à maillage en nylon (21, 23) fixée à ladite bague souple et contenant intérieurement un noyau en mousse souple (15) ; et
ledit au moins un élément de retenue souple (18) est constitué par une longueur discrète d'un fil métallique enroulé, fixé suivant une partie de sa longueur audit diaphragme (14).

13. Prothèse d'obturation souple et système de mise en place par transcathéter selon la revendication 9, dans lesquels ledit au moins un filament (30) comprend :
une bague (34) de traction, ladite bague de traction (34) ayant un diamètre plus grand que ledit cathéter pousseur (28) pour empêcher qu'elle passe à travers lui ;
ledit au moins un élément souple de retenue (18) comprend une ouverture et ledit filament (30) passe à travers l'ouverture ;
ledit filament (30) est connecté à une extrémité audit cathéter pousseur (28) et une autre extrémité à ladite bague de traction (43) ; et
dans lesquels, lorsque l'on tire sur ladite bague de traction (34), on provoque la déformation par lesdits filaments (30) dudit élément de retenue (18).

14. Prothèse d'obturation souple et système de mise en place par transcathéter selon la revendication 13, dans lesquels lesdits filaments (30) sont des monofilaments constitués de nylon.
